# EUROPEAN PATENT APPLICATION

(11) **EP 3 750 641 A1**
(43) Date of publication of application: **16.12.2020**
(21) Application number: 19020382.8
(22) Date of filing: 13.06.2019
(51) Int. Cl.: B08B 3/10, C11D 11/00, A61L 2/18, B29B 17/00

(54) **A METHOD FOR REMOVING ODORS FROM SOLID MATERIALS AND A DEVICE FOR CARRYING OUT SAID METHOD**

(71) Applicant: Linde GmbH, 82049 Pullach (DE)
(72) Inventor: Hanke, Ulrich, 82041 Oberhaching (DE); Domanski, Stephanie, 80799 München (DE)
(74) Representative: Gellner, Bernd

(57) **Abstract**

The invention relates to a method for removing odorants from solid materials (6) by which a solid material (6) is washed with water (3), with additional and simultaneous application of ozone (2) to the solid material (6). Furthermore, the invention relates a device (50) for carrying out the method.

## Description

The invention relates to a method for removing odors from solid materials and a device for carrying out such a method.

### Prior art

Plastic waste is recycled to produce functional and useful products from scrap materials and to reduce pollution caused by plastic production and consumption. Recycling plastic also reduces the amount of plastic that is sent to landfills or the energy consumed during plastic incineration. It also reduces the need for additional raw materials needed to produce plastic from virgin materials.

The plastic recycling process involves the following basic steps: collection, sorting, size reduction, cleaning/washing, melting and forming of the final product. Additional separation steps can also be applied to further separate polymer types and colors. During the recycling process, odorants and contaminants shall also be reduced to ensure the quality of the plastic product produced.

Well-known odor removal methods include contacting the formed product with hot gas, stripping volatile substances and adding virgin polymer materials. For example, hot air is blown over the formed product to remove odors.

Alternatively, it is known to blow air containing gaseous ozone onto plastic materials or rubber to remove odors. Gaseous ozone is also used to remove odors from the interior of used vehicles. In "Wypich, Handbook of Odors in Plastic Materials (2nd Edition), 2017" a number of methods for removing odors are mentioned. However, residues can also remain here.

With the increasing demand to recycle plastic waste materials, new methods are required to minimize odor problems in plastic products. Removing odorants from plastic waste is a key challenge to recycle and up-cycle plastics and achieve closed-loop recycling.

There are numerous potential sources of odorants in plastic waste, including but not limited to, the migration of odorants and other contaminants from filling, residual byproducts such as solvents, environmental factors such as heat or radiation, chemical degradation such as fat oxidation and microbial contaminants.

Studies have shown that odorants or aroma compounds such as aldehydes, ketones, carboxylic acids and phenols contribute to odors found in plastic waste materials.

Current plastic recycling methods do not always efficiently remove odorants. If odors are not successfully removed, a product may be less valuable and downgraded. Downgrading or secondary recycling refers to the reprocessing of plastic waste into products with lesser properties.

By achieving a process where closed-loop recycling may be achieved, this reduces the requirement for plastic to be produced from virgin materials, which contributes to combatting plastic pollution.

Therefore, further improvement possibilities are sought for the removal of odors in the recycling of plastic materials.

### Disclosure of the invention

The invention proposes a method for removing odors and a device for carrying out such a method in accordance with the independent patent claims. Advantageous embodiments result from the respective subclaims and the following description.

### Advantages of the invention

According to a first aspect of the invention, a method is proposed in which a solid material is washed with water, with additional and simultaneous application of ozone to the solid material, in other words, ozone comes into contact with the solid material together with the water.

Ozone is a powerful oxidant that can selectively oxidize the odor causing compounds found in plastic waste. Introduction of ozone into water significantly increases odor removal compared to dry ozone exposure in air. Odors are removed more efficiently and in a shorter contact time compared to other air-based or chemical oxidation processes.

In an advantageous embodiment, simultaneous exposure of the solid material to water and ozone lasts at least 10 seconds, preferably 10-30, more preferably about 15 seconds. Within 15 seconds a wet application of ozone can typically remove 98% of the odors of polymer materials. A dry application of ozone only achieves 80%.

It is preferable to inject the ozone into the water before or during the washing process. It is imaginable that water is taken from a washing chamber for washing the solid material, pumped through a ring line comprising a Venturi nozzle, by means of which ozone is introduced and fed back into the washing chamber. This is an easy option to introduce ozone into the washing water. It can be used to remove odors during the washing process.

Alternatively or additionally preferred, the ozone is added to a washing chamber during the washing process. Preferably, a gas-tight chamber is used with a leakage rate of 10⁻⁰⁷ mbar l/s or less. This can be advantageous because comparatively more ozone can be added than if the ozone is dissolved exclusively in water.

In an advantageous embodiment, the solid material is exposed to water and ozone after an initial cleaning or washing stage. The washing process utilizing water and ozone is carried out in a separate chamber to the initial cleaning or washing stage to ensure any contaminants washed in the initial stage are already removed before the addition of ozone.

Preferably the solid material is a plastic material, preferably plastic waste to be recycled. As mentioned at the beginning, a solution is particularly urgent for these materials. Furthermore, the application to plastic waste differs from the prior art in which attempts to remove odors are carried out on the finished end products, for example by blowing a hot gas onto a granulate product.

The advantages and improvements achieved by the wet use of ozone, i.e. the combined use of ozone and water to remove odors from plastic waste for plastic recycling, are as follows:
- increasing the value of the finished plastic product by ensuring more efficient odor removal;
- the potential for faster processing, as the ozone is applied to the solid material during the washing process and reacts rapidly with the odorous substances, while the current odor removal process is carried out after the product has already been manufactured;
- the improvement of the plastics recycling process and the avoidance of plastics being sent to landfills or incinerators; thus also a reduction in plastics pollution;
- achievement of a closed recycling cycle; thus a reduction of the need to produce new plastic from virgin materials.

According to another advantageous embodiment, the solid material is crushed before washing. By increasing the surface area, the odor can be removed even more efficiently during the washing process.

Expediently, a quantity, temperature and/or pressure of the added ozone are checked during washing. In this way, the settings for the above values can be optimized for efficient odor removal.

A preferred ozone concentration of more than 2 mg/l up to a maximum of 150 mg/l, is introduced into the wash water in order to effectively remove odorous compounds from the solid material. Maximum solubility of ozone in water at 5°C and 1 bar is 150 mg/l. A significant odor reduction has already been measured for concentrations as low as 5 mg/l.

In particular, the temperature of the added ozone is preferably between 10 and 30 °C. A temperature below 30°C ensures that no decomposition of ozone due to high temperatures occurs. An advantageous temperature is about 15-20°C, preferably 17°C. A pressure of the added ozone between 1.5 and 4 bar is advisable. A pressure just a little over 1 bar is sufficient in order to introduce ozone through typical vents or a venture.

In another advantageous embodiment, a temperature and/or pressure in a washing chamber is controlled during washing. This also makes it possible to further optimize the settings for these values for efficient odor removal.

In particular, a temperature in the washing chamber is preferably between 5 and 95 °C, more preferably between 5 and 65 °C and most preferably between 5 and 35 °C. A higher temperature would lead to ozone decomposition. A lower temperature would lead to low ozone solubility.

Expediently, a pressure in the washing chamber is equal to the atmospheric pressure. This is preferable, because in this way no gas-tight chamber is required.

According to a second aspect of the invention, a device for carrying out a method according to the first aspect of the invention is proposed, comprising a washing chamber, an ozone supply and a control unit equipped to carry out the process.

Further improvements of the invention by identifying the ideal process conditions and process steps for ozone supply and oxidation of odorous substances to avoid plastic degradation are conceivable within the scope of the patent claims.

Further advantages and embodiments of the invention result from the description and the enclosed drawings.

It is understood that the features mentioned above and the features to be explained below can be used not only in the combination indicated, but also in other combinations or in a unique position, without leaving the scope of this invention.

The invention is shown schematically in the drawing using an example and is described below with reference to the drawing.

### Short description of the drawing

- Figure 1: shows a schematic side view of an embodiment of a device according to the invention.
- Figure 2: shows a schematic side view of another embodiment of a device according to the invention.
- Figure 3: shows a schematic flow chart of an embodiment of a device according to the invention.

### Detailed description of the figures

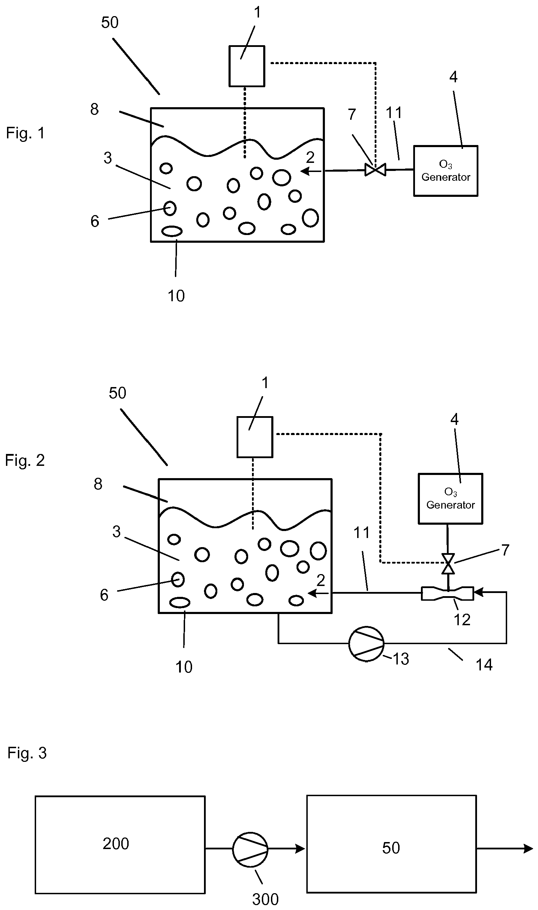

Figure 1 shows a schematic representation of a device according to the invention, marked 50.

The device for carrying out a method conforming to the invention has a control unit 1 which is arranged to carry out an embodiment of a method conforming to the invention. Furthermore, the device 50 has a washing chamber 10 and an ozone supply 4. The washing chamber 10 is filled with water 3 and there are particles of solid material 6 in the water 3, which are washed in it. Ozone 2 is injected into the water in the washing chamber and is dissolved into water 3. The ozone connection 4 is connected to an ozone generator 4. Above the water there is an air atmosphere 8.

The solid material particles 6 in this design consist of plastic waste that has been previously shredded. The water tank 10 may have a rotating device designed to rotate the washing chamber 10 or the water inside it to carry out a washing operation.

In an embodiment of the inventive method carried out with this device 50, sorted plastic waste 6 is put into the water 3 in the washing chamber 10. Then the washing chamber 10 is closed. Through the ozone connection 11, ozone 2 is then added to the water container 10 via a valve 7 by means of the control unit 1 dependent on the ozone concentration in the water 3, and the divided plastic waste 6 is washed by the ozone-containing water 3. This washing process is carried out for at least 15 seconds so that odorants are removed from the plastic waste.

Figure 2 shows a schematic side view of another embodiment of a device according to the invention. In this embodiment the ozone 2 is introduced by means of a venturi nozzle 12 installed in a ring line 14. By means of a pump 13 the water is taken from the chamber 10, pumped through the venturi nozzle 12 and fed back to the chamber 10.

Figure 3 shows a schematic side view of an embodiment according to the invention in which the ozone treatment device 50 is arranged downstream of an initial cleaning or washing chamber 200. The precleaned material can be transported between the two cleaning chambers by means of a conveyor screw or pump 300.

## Claims

1. A method for removing odors in solid materials (6), in which a solid material (6) is washed with water (3), wherein ozone (2) is additionally and simultaneously applied to the solid material (6).

2. The method according to claim 1, wherein the ozone (2) is injected into the water (3) before or during the washing process.

3. The method according to claim 1 or 2, wherein the ozone (2) is added to a washing chamber (10) during the washing process.

4. The method according to claim 2 or claim 3, wherein the water (3) is taken from a washing chamber (10) for washing the solid material (6), pumped through a ring line (14) comprising a venture nozzle (12), by means of which ozone is introduced and fed back into the washing chamber (10).

5. The method according to any one of the above claims, wherein the solid material (6) is a plastic material, preferably a plastic waste to be recycled.

6. The method according to any one of the above claims, wherein the solid material (6) is shredded before washing.

7. The method according to any one of the above claims, wherein the solid material is exposed to water and ozone after an initial cleaning or washing stage, wherein the washing process utilizing water and ozone is carried out in a separate chamber to the initial cleaning or washing stage.

8. The method according to any one of the above claims, wherein a simultaneous exposure of the solid material (6) to water (3) and ozone (2) lasts at least 10, preferably 10-30, more preferably about 15 seconds.

9. The method according to any one of the above claims, wherein during washing a dose, a temperature and/or a pressure of the added ozone are controlled.

10. The method according to claim 9, wherein an ozone concentration of more than 2 mg/l up to a maximum of 150 mg/l is introduced into the wash water.

11. The method according to claim 9 or 10, wherein a temperature of the added ozone is preferably between 10 and 30 °C.

12. The method according to any one of claims 9 to 11, wherein a pressure of the added ozone is preferably between 1.5 and 4 bar.

13. The method according to any of the above claims, wherein during washing a temperature and/or a pressure in a washing chamber are controlled.

14. The method according to claim 13, wherein a temperature in the washing chamber is between 5 and 95 °C, preferably between 5 and 65 °C and more preferably between 5 and 35 °C.

15. A device (50) for carrying out a method according to any one of the above claims, which has a washing chamber (10), an ozone supply (4) and a control unit (1) which is set up to carry out the method.
